# EUROPEAN PATENT APPLICATION

(11) **EP 2 377 526 A1**
(43) Date of publication of application: **19.10.2011**
(21) Application number: 10305288.2
(22) Date of filing: 23.03.2010
(51) Int. Cl.: A61K 9/70, A61K 31/00, A61K 31/403, A61K 31/715

(54) **Fast dissolving drug delivery systems**

(71) Applicant: BioAlliance Pharma, 75015 Paris (FR)
(72) Inventor: Constantini, Dominique, 75014 Paris (FR)
(74) Representative: Jacobson, Claude

(57) **Abstract**

The present invention related to the administration of bioactive agents via oral fast dispersing/dissolving drug delivery systems for treating illnesses of patient with critical buccal condition, leading to difficulties in swallowing oral medicine forms and thus difficulties in treating said illnesses. Particularly, the present invention discloses the administration of bioactive agents via oral fast dispersing/dissolving drug delivery systems for treating illnesses of patient with dysphagia and/or odynophagia and/or aspiration risk. Another aspect of the present invention is the administration of bioactive agents via oral fast dispersing/dissolving drug delivery systems for the treatment of dysphagia and/or odynophagia and/or aspiration risk.

## Description

### TECHNICAL FIELD OF INVENTION

The present invention related to the administration of bioactive agents via oral fast dispersing/dissolving drug delivery systems for treating illnesses of patient with critical buccal condition, leading to difficulties in swallowing oral medicine forms and thus difficulties in treating said illnesses. Particularly, the present invention discloses the administration of bioactive agents via oral fast dispersing/dissolving drug delivery systems for treating illnesses of patient with dysphagia and/or odynophagia and/or aspiration risk. Another aspect of the present invention is the administration of bioactive agents via oral fast dispersing/dissolving drug delivery systems for the treatment of dysphagia and/or odynophagia and/or aspiration risk.

### BACKGROUND OF INVENTION

Some patients experience critical buccal conditions, involving difficulty in swallowing tablets and capsules and thus leading to problems in administration of oral medicine forms. These critical buccal conditions are known as "dysphagia", a general term meaning difficulty in swallowing, or more particularly "odynophagia", meaning a painful swallowing or "aspiration risk", when material such as medicine, food, saliva, or nasopharyngeal secretions are inhaled into the airway or respiratory tract instead of digestive tract.

Dysphagia, or difficulty in swallowing, is a condition or symptom, which can be caused by many different conditions, injuries or diseases. Dysphagia can cause choking, aspiration of food or liquid into the lungs, which can lead to pneumonia (called "aspiration pneumonia") or incomplete administration of oral medication. These factors can at least discomfort a patient, but may also interfere with or complicate conventional treatment of patients when treating other coexisting disorders. Among the many contributions to or causes of dysphagia are head injuries, brain stem impairment, cerebral vascular accidents, Parkinsonism, Alzheimer's disease, muscular dystrophy, cerebral palsy, cancer (notably brain tumors), diabetes, medication side effects, advanced HIV infections, gastrointestinal disorders, gastro esophageal or reflux disorder. It has been estimated that more than 10,000 patients a year choke to death as a result of dysphagia.

Patients with cancer under chemotherapy or radiotherapy, or post-operative patients are very often suffering from side effects such nausea or vomiting which has to be addressed. But due to side effect of chemotherapy or radiotherapy, these patients present critical buccal conditions, such as dysphagia or difficulties to swallow. Furthermore, ondansetron, one of the most recommended bioactive agent for the treatment of nausea or vomiting, is known as a drug inducing dysphagia in long-term treatment. In this case, there is therefore a particular need to identify a solution suitable for administering ondansetron and to limit the risk of disphagia.

Patients with head/neck injuries or AIDS are also often concerned by dysphagia.

Such conditions are often experienced by older patients. As the population ages, elderly people suffering from dysphagia are increasing. Many elderly people may have difficulty in swallowing foods and develop symptoms of dysphagia. In many cases, dysphagia may cause aspiration, and the entry of the aspirated substance into the lung may cause bacterial infection, resulting in pneumonia.

It is also a common experience with pediatric patients who tend to be non compliant with the administration of solid oral dosage forms. Often, dysphagia is induced by drugs used in a long-term treatment to treat other disorder. It is the case, for example, for the nervous central system drugs, cardio-vascular drugs or anti-inflammatory and analgesic drugs. Furthermore, children are at highest risk for death and injury from mechanical airway obstruction, due in large part to their immature anatomy (Tarrago, 2000). They do not have fully developed teeth for chewing, and lack molars. In addition, their airways are small, they have less chewing capacity, and their respiratory rate is high compared to older children and adults (Gregori, 2008). Therefore, aspiration is a real risk for children.

Dysphagia can often be improved thanks to hygiene and dietetic methods or bioactive agent such as proton pump inhibitors (PPI), anti-H2 or prokinetic agents. However, these medications involve secondary effects (iatrogenic pathologies) and long-term usage must be avoided.

PPIs are very strong inhibitors of the acidic stomach secretion. They are particularly indicated in the treatment of erosive oesophagitis or peptic stenosis. They could also be used on a long term basis but they have strong iatrogenic effects (oligoptyalism, Table II) and are also responsible for patient's confusion. For the elderly over 80, they should not be used too long. These are omegrazole, lanzopra-zole and pantoprazole.
Anti-H2 are efficient on 60% of the symptoms and efficiency decreases with time. These are cimetidine, ranitidine, famotidine or nizatidine. They also have strong iatrogenic effects, notably oligoptyalism and must be handled with precaution in patients who have a decrease of creatine clearance, which is more and more frequent with the increase in age.
Prokinetic agents are as effective as anti-H2s but have the same drawbacks and more side effects (diarrhea, nausea, medication interactions).

When dysphagia is due to drugs inducing dysphagia in long-term treatment, a solution is to suppress such medications. This is unfortunately not always possible and depends on the gravity of the illness.

Conventional oral dosage forms, such as tablets, pills, caplets, or capsules, are designed for short residence time in the mouth. Absorption of the active agent from these dosage forms occurs in the gastrointestinal tract only after the dosage form undergoes disintegration followed by dissolution of the agent in the gastric fluids. These dosage forms are thus not suitable for patients with dysplagia or difficulties to swallow.

To address the issue of oral administration of bioactive principle in patient presenting difficulty to swallow, delivery of drugs in a liquid dosage form has been developed. Indeed, liquid form is more convenient to be swallowed than solid form. However, handling and delivering said liquid system is always a challenge compared to solid system. Effectively, liquid dosage form often presents instability when stored and it is more difficult to precisely dose the bioactive agent when administered to patients. Therefore, dosage forms with the convenience of liquids and storage ability and dose precision of solid oral dosage form is desirable.

However, liquid form is still unsuitable to patient presenting severe difficulties to swallow.

To overcome this drawback, tablets have been formulated to be quick dispersed in the mouth. The bioactive agent thus rapidly dissolves in the saliva to provide a liquid formulation which is very easily swallowed. However, the oral disintegration/dispersion time appears to be a critical feature to solve the drawback of the art. Indeed, oral disintegration/dispersion time of current disintegrating/dispersing tablet often take few minutes, which is too long to overcome a risk associated with choking or gagging that occurs with subjects having limited control of their swallowing reflexes. For children, it is often advised to preliminary dissolve the tablet with water, but again not suitable in case of difficulties to swallow.
Moreover, it is sometimes advised to add water to accelerate the disintegrating/dispersing of the tablet in the mouth. But such added water is difficultly swallowed by patient with critical buccal condition.

In addition, several manufacturers have proposed formulations that could be used to deliver bioactive agents. The vast majority of these formulations are "mucoadhesive" formulations designed for adhesion of the dosage form to mucosal tissue in the mouth, and transmission of the drug from the dosage form through the mucosal tissue into the systemic circulation. As described in U.S. Patent No. 6,750,921 to Kim et al., film-forming agents have been used to manufacture drug delivery formulations for percutaneous or transdermal application, but these necessarily involve an adhesive composition to retain the agent in situ long enough to cause sustained release of the active ingredient. Bioerodible films are described in Tapolsky et al., U.S. Patent No. 5,800,832. The films have an adhesive layer and a non-adhesive backing layer and are intended to adhere to the mucosal surface. Biegajski et al., U.S. Patent No. 5,700,478, describes a water-soluble pressure-sensitive mucoadhesive suitable for use in a mucosal-lined body cavity. The purported advantage of these mucoadhesive films resides in their transmucosal route of absorption and thus their ability to bypass the gastrointestinal tract, and barriers in the gastrointestinal tract to drug absorption such as first pass metabolism and decomposition of the active ingredient in the stomach. However such a delivery system targets a new route of administration and therefore cannot be use for bioactive agents for which their clinical efficacy has been proven for oral administration. Indeed a particularly advantageous delivery system would be formulated or administered for gastrointestinal absorption of the bioactive agent, and that are bioequivalent to and interchangeable with existing orally administered drug products.

Therefore what is needed is a solid, non mucoadhesive and fast dissolving without added water delivery system for oral administration to patient presenting critical buccal condition.

### SUMMARY OF THE INVENTION

The present invention relates to film dosage forms that are formulated or administered for gastrointestinal absorption of the bioactive agent, and that are bioequivalent to and interchangeable with existing orally administered drug products. These film dosage forms are non-mucoadhesive; and they are absorbed predominantly through the gastrointestinal tract. Most importantly, these dosage forms are specially formulated to meet exacting bioavailability requirements, or to be bioequivalent to existing orally administered dosage forms and which is particularly suitable for patients with dysphagia as they quickly disintegrate in the mouth when exposed to saliva, and does not need added water to accelerate its disintegration.

Therefore, in a first principal embodiment, the present invention provides an oral fast dissolving film comprising an active principle for treating illnesses of patients with critical buccal condition. Added water being not required to facilitate the dissolution or swallowing of the active principle.

In preferred aspects of the invention critical buccal condition is dysphagia, aspiration risk, odynophagia, chemotherapy and/or radiotherapy-induced mucositis, head and neck or oesophageal cancer, or reduced salivary flow.

A further preferred object of the invention is an oral fast dissolving film comprising an active principle for treating illnesses such as, neuropathy diseases (Alzheimer diseases, Parkison diseases, epilepsy, ...) or lung diseases. A more preferred object of the invention is an oral fast dissolving film comprising an active principle for treating illnesses of children or elderly. Patient with iatrogenic pathologies are also preferred. As usually in geriatrics, iatrogenic pathologies are often implicated. Some drugs may for instance induce alivary secretion impairments (antidepressants, tricyclics; anticholinergics...), even alterations of the oropharyngeal sensation, (toxins...).

The oral fast dissolving film of the invention is also suitable to treat a patient with one or more of the following bioactive principle, inducing oligoptyalism in long-term treatment:

| **Drugs for the nervous central system** |
|---|
| **Anxiolytics and hypnotics** |
| |
| **Benzodiazepines:** alprazolam (Xanax^{®}), bromazépam (Lexomil^{®}), Clorazepate (Tranxene^{®}), Iorazépam (Temesta^{®}), flunitrazépam (Rohypnor^{p}) |
| |
| **Others:** hydroxyzine (Atarax^{®}), zopiclone (Imovane^{®}), acéprométazine (Meprozine^{®}, Noctran^{®}) |
| |

| **Antidepressive** |
|---|
| |
| **Imipraminics:** clomipramine ((Anafranil^{®}), imipramine (Tofranil^{®}), amiptriptlyline (Laroxyl^{®}), trimipramine (Surmontil^{®}), maprotiline (Ludiomil^{®}) |
| |
| **Serotoninergics:** fluvoxamine (Floxyfral^{®}), fluoxetine (Prozac^{®}), paroxetine (Deroxatn, sertraline (Zoloft^{®}), citolopram (Séropram^{®}) **IMAO**: moclobémide (Moclamine^{®}), toloxatone (Humoryl^{®}), iproniazide (Marsilid^{®}) |
| |
| **Others:** tianeptine (Stablon^{®}), milnacipran (Ixel^{®}), venlafaxine (Effexor^{®}), miansérine (Athymil^{®}) |
| |

| **Neuroleptics** |
|---|
| |
| **Phenothiazines:** chlorpromazine (Largactil^{®}), levome romazine (Nozinan^{®}), cyamemazine (Tercian ^{®}), thioridazine (Mellerr), propericiazine (Neuleptil^{®}), thiopreperazine (Majeptil ), pipotiazine (piportil^{®}) |
| |
| **Butyrophenones:** halopéridol (Haldol^{®}), droperidol (Droleptan^{®}), pimozine (Orap^{®}), pipampérone (Dipiperon^{®}) **Thioxanthenes:** flupentixol (Fluanxol^{®}), zuclopenthixol (clopixol^{®}) |

| **Cardio-vascular drugs** |
|---|
| **Antiarythmics:** disopyramide ( Rythmodan^{®}, Isotythm LP), flecabide (Flecaine^{®}) |
| |
| **Central antihypertensive:** rilmenidine (Hyperium^{®}), moxinidine (Physiotens^{®}), clonidine (Catapressan^{®}, Clonidine^{®}), methyldopa (Aldomet^{®}), guanfacine (Estulic^{®}) |
| |
| **Alpha-1 blockers:** urapidil (Eupressyl^{®}, Mediatensyl^{®}), prazosine (Minipress^{®}, AlpressLP) |
| |
| **Betablockers:** acebutolol (Acebutol^{®}, Sectral^{®}), atenolol (Atenolol^{®}, Tenormine^{®}, Betatop^{®}), betaxolol (Kerlone^{®}), bisoprolol (DetensieV, Soprol^{®}), carvedilol (Kredex^{®}), tabetalol (Trandate^{®}), metoprolol (Lopressor^{®}, Seloken^{®}), nodalol (Corgard^{®}), pindolol (Visken^{®}), propanolol (Propanolol^{®}, Avlocardyl^{®}), sotalol (Sotalex^{®}), timolol (Timacor^{®}) |
| |

| **Calcic blockers:** |
|---|
| |
| **Bradycardising:** diltiazem (Tildien^{®}, Bi-Tildiem^{®}, Dilt azel^{®}, Mono-Tildiem^{®}, Cardiosta LP), bépridil (Unicordium^{®}), verapamil (verapramil LP, Isoptine LP) |
| |
| **Dihydropyridines:** amlodipine (Amlor^{®}), felodipine (Flodil LP), isradipine (Icaz LP), nicardipine (Loxen LP), nitrendipine (Baypress^{®}. NifreV), nifedipine (Nifedipine LP, Adalate LP, Chronadalate LP) |
| |

| **Diuretics** |
|---|
| |
| **Central:** furosemide (Lasilix^{®}, Furosemide^{®}), bumetanide (Burinex^{®}), piretanide (Eurelix LP) |

| **Anti-inflammatory and analgesic drugs** |
|---|
| **Analgesics** |
| |
| **Major:** morphin (Morphine LP. Moscontin LP, Skenan LP, Kapanol LP), buprenorphin (Temgesic^{®}), nalbuphine (Nubain^{®}). pentazocine (Fortal ), pethidin (Dolosal^{®}) |
| |
| **Minor:** codein (Codein Efferalgan^{®}, Codein Dafalgan^{®}, Lindilane^{®}, Codoliprane), dihydrocodeine (Dicodin LP), |

### Other drugs

Antiemetics: metoclopramide (Primpéran^{®}, Anausin LP, Prokinyl LP), metopinazine (Volgalene^{®}), ondansetron (Zophren^{®}), diphenhydramine (Nautamine^{®}), dimenhydrinate (Nausicalm^{®}, Dramamine^{®}), scopolamine (Scopoderm Tts)
Antihistaminics: méquitazine (Primatan^{®}), cétirizine (Virlix^{®}, Zyrtec^{®}), ioratidine (Clarityne^{®}), prométhazine (Phénergan^{®}), aliméthazine (Théralène^{®}), dexchlorphéniramine (Polaramine Repetabs, Polaramine), oxatomide (Zaditen LP)

### Antispasmodics

Non musculotropic: tiemobium (Visceralgine^{®}), dihexyver ne (Spasmodex^{®})
Musculotropic: alverine (Spasmaverine^{®}, Meteospasmyl^{®})

### ENT and bronchial dilators

Decongestionning: with pseudoephedrin (Sudafed^{®}, Rhinadvil^{®}, Rhinureflex^{®}), phenylpropanolamin (Rinurel^{®}, Rinufan^{®})
Bronchodilatators: salbutamol (Ventoline^{®}), terbutalin (Bricanyl^{®}), pirtuberol (Maxair^{®}), formoterol (Foradil^{®}), ipatropium (Atrovent^{®}, Combivent^{®}. Bronchodual^{®}), oxitropium (Tersigat^{®})
Urology (Alpha-I-Blockers): alfuzosine (Xantal LP, Urion^{®}), tamsulosine (Josir LP, OmixLP)
Anti-ulcerous: sucrafalte (Keal^{®}, Ulcar^{®}), iansoprazole (Lanzor^{®}, Ogast^{®}), omeprazole
(Mopral^{®}, Zoltum^{®}), famotidine (Pepdine^{®}) Anti-cancerous: busufan (Myleran^{®}), procarbazine (Natulan^{®})
Anti-infectious: griseofuline (Fulcine Forte, Grisefuline^{®}), flucytosine (Ancotil^{®}), metronidazole (Flagyl^{®}), pyrimethamine (Malocide^{®}, Fabsidar^{®}), enoxacin (Exonor^{®}), norfloxacin (Noroxine^{®}), ofloxacin (0flocet^{®}), ciprofloxacin (Ciflox^{®}), didanosin (Videx^{®}), zalcitabin (Hivid^{®}), ganciclovir (Cymcan°)

Another object of the present invention is to provide an oral fast dissolving film comprising an active principle for the treatment of dysphagia, aspiration risk, odynophagia, chemotherapy and/or radiotherapy-induced mucositis, head and neck or oesophageal cancer, or reduced salivary flow.

In another preferred embodiment, the bioactive agent to treat the dysphagia is proton pump inhibitors (PPI), anti-H2 or prokinetic agents.

In a further preferred embodiment, the bioactive agent is suitable to treat illnesses despite its adverse effect to induce dysphagia with long-term treatment.

A particularly preferred drug of the present invention is ondansetron and more preferably ondansetron base.

In a further aspect, the oral fast dissolving film without water of the invention comprises an bioactive agent, a hydrophilic binder and a water-soluble diluents.

In a preferred embodiment, the bioactive agent is selected from: donepezil hydrochloride; ondansetron base; desloratadine; olanzapine; risperidone; rivastigmine tartrate; sildenafil; vardenafil; galantamine; diclofenac potassium; buprenorphine HC1; naloxone HC1 dehydrate; alprazolam; clonazepam; diazepam; lorazepam; sumatriptan; eletriptan; rizatriptan; zolmitriptan; naratriptan; almotriptan; frovatriptan; cetirizine hydrochloride; loratadine; ambroxol hydrochloride; apomorphine; ascorbic acid; betamethasone; caffeine; dextromethorphan; glimepiride; hydrocortisone; ketotifen; loperamide; meclozine; melatonin; neramexane; piroxicam; sodium picosulfate; and zinc histidine; or a pharmaceutically acceptable salt thereof; or a combination thereof.

Furthermore, the oral fast dissolving film without water of the invention comprises from 0.05% to 50% by weight of said bioactive agent.

The oral fast dissolving film without water of the invention also comprises from 40% to 80% by weight of one or more ingredients that constitute a hydrophilic binder and a water soluble diluent, the hydrophilic binder can be Polyvinylalcohol and the water-soluble diluents can be rice starch.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DETAILED DESCRIPTION OF THE INVENTION

The present invention may be understood more readily by reference to the following detailed description of preferred embodiments of the invention and the Examples included therein.

### Definitions

As used in this specification and in the claims which follow, the singular forms "a,", "an" and "the" include plural referents unless the context clearly dictates otherwise. Thus, for example, reference to "an ingredient" includes mixtures of ingredients, reference to "an active pharmaceutical agent" includes more than one active pharmaceutical agent, and the like.

The term "disintegrate" has its usual and customary meaning in the pharmaceutical arts, as described in <701> of the U.S. Pharmacopoeia (2005 USP/NF) for uncoated tablets, using a basket rack assembly operating at 30 cycles per minute through a distance of 5.5 cm, in a disintegration medium at 37°C. When disintegration requirements are discussed herein, they are preferably met under the foregoing testing conditions, at a pH of 4.0 or 6.8. A film or other dosage form is said to be "disintegrated" if it is completely disintegrated, a state in which any residue of the unit remaining on the screen of the test apparatus, or in the mouth, is a soft mass having no palpably film core, or fragments of a tablet coating or capsule shell. Disintegration thus does not imply complete dissolution of the dosage unit or even the active constituent, although a dissolved dosage unit would typically be completely disintegrated.

When reference to Ph. Eur. 2.9.1 (disintegration) is made herein, it will be understood that thedisintegration conditions described above under <701> USP can also be employed.

The term "dissolution" also has its usual and customary meaning in the pharmaceutical arts, as described in <711> and <724> of the U.S. Pharmacopoeia (2005 USP/NF). Therefore, a film is said to be "dissolved" if, upon testing by the method of U.S. Pharmacopoeia (2005 USP/NF), the amount of active agent dissolved in the dissolution medium exceeds a predetermined percentage. When dissolution conditions are given, it will be understood that stirring preferably occurs in 0.1N hydrochloric acid buffer (pH=2), or at pH 1.2, pH 4.0 or 6.8, at 37° C, using the paddle method at 50 rpm in a type II dissolution apparatus.

A "orally dissolving or orally dispersible tablet" ("ODT") refers to an uncoated tablet intended to be placed in the mouth where it can disperse rapidly before being swallowed, as described in Eur. Ph. 5.0. An ODT disintegrates within three minutes when tested according to the disintegration testing described herein.

The term "non-mucoadhesive" means that the dosage form is not designed for administration of the active pharmaceutical agent through the oral mucosa. I.e. the dosage form is not designed to adhere to the mucosal surfaces of the buccal cavity as an intact film or disintegrated film residue.

The term "absolute bioavailability" refers to the availability of the active drug in systemic circulation after non-intravenous administration (i.e., after oral, rectal, transdermal, subcutaneous administration). In order to determine absolute bioavailability of a drug, a pharmacokinetic study must be done to obtain a plasma drug concentration versus time plot for the drug after both intravenous (IV) and non-intravenous administration. The absolute bioavailability is the dose-corrected area under curve (AUC) non-intravenous divided by AUC intravenous.

When pharmacokinetic parameters are given herein (i.e. T max, absolute bioavailability, etc.), it will be understood that they can refer to the mean, median, or individual observed pharmacokinetics, and that mean pharmacokinetics are intended when claimed unless stated to the contrary.

Unless specified otherwise, the term "wt. %" as used herein with reference to the final product (i.e., the film, as opposed to the formulation used to create it), denotes the percentage of the total dry weight contributed by the subject ingredient. This theoretical value can differ from the experimental value, because in practice, the film typically retains some of the water and/or ethanol used in preparation.

When doses are given for a drug and its salt, it will be understood that the calculated dose is based on the molecular weight of the active pharmaceutical ingredient, which includes the cationic and anionic species in the case of a salt, and just the base when the active principle is not present as a salt. In addition, when reference is made to the salt of a drug and pharmaceutically acceptable salts thereof, it will be understood that salts of the base form of the base drug are intended.

"Critical buccal conditions" means a patient with a dysphagia, odynophagia, aspiration risk oropharyngeal candidiasis, herpes, mucositis, severe aphteous and/or lichen planus

"Fast dissolving" or "flash dissolving" means a dissolving within less than 60 seconds and more preferably within less than 30 seconds.

### Discussion

As discussed above, the invention provides a physiologically acceptable film that is particularly well adapted to disintegrate rapidly when placed on the tongue of a patient, and to facilitate gastrointestinal absorption of the pharmaceutically active agent. The film and bioactive agent need not dissolve entirely in the mouth. The film could be not entirely dissolved.

When tested according to Ph. Eur. 2.9.3, paddle over disc, the film preferably dissolves (at least 80% or 100% active agent release) within about 15, 10 or 5 minutes, when tested at pH 1.2, 4.0 or 6.8.

The film may also be characterized by the time it takes to disintegrate completely, and it preferably disintegrates to a soft residue within about 10, 20, 30 or 60 seconds of administration, after which it is swallowed. These disintegration times are preferably observed in the oral cavity when the film is administered, as well as when tested for disintegration using the method described in Ph. Eur. 2.9.1. The prompt disintegration and swallowing of the film helps to assure gastrointestinal absorption of the dosage form. The film is not of the conventional mucoadhesive type, designed to deliver active agent transmucosally.

### Film Formulation

Preferred films according to the invention comprise a pharmaceutically active agent, a film-forming agent, and at least one of the following additional ingredients: water, antimicrobial agents, water soluble diluents such as plasticizing agents, softeners, and fillers, flavoring agents, saliva stimulating agents, cooling agents, stabilizers, surfactants, stabilizing agents, emulsifying agents, thickening agents, binding agents, coloring agents, sweeteners, fragrances, triglycerides, preservatives, polyethylene oxides, propylene glycol, and the like. In a preferred embodiment, the film comprises one or more ingredients that act both as water soluble binding agents and hydrophilic polymers, such as polyvinyl alcohol, polyethylene glycol ("PEG"), propylene glycol, polyethylene oxide, and starches, celluloses, gelatines and the like. Therefore, when it is stated herein that a formulation comprises a water soluble binding agent and a hydrophilic polymer, it will be understood that these two agents may be describing one solitary ingredient. The finished film product preferably comprises from about 40 to about 80 wt.% of these ingredients, and more preferably from about 50 to about 75 wt.%. The active agent preferably makes up from 5 to 20 wt.% of the finished film formulation, more preferably from about 8 to about 15 wt.%. The formulation is also preferably "surfactant free." Alternatively, the formulation may contain one or more surfactants.

A preferred taste masking agent, which facilitates the dissolution of the product, and it is believed helps to maintain the amorphous state of certain active ingredients such as donepezil, is an aminoalkyl methacrylate copolymer such as that marketed as Eudragit E PO. The aminoalkyl methacrylate copolymer preferably contains diethyaminoethyl residues, and preferably comprises from about 20 to about 26 wt.% of such groups in a dry substance basis.

The average molecular weight of the copolymer preferably ranges from about 120,000 to about 180,000, or from about 140,000 to about 160,000, most preferably about 150,000. Preferred methacrylic monomers include butyl methacrylate and methyl methacrylate. This agent is preferably present in the final film in an amount of from about 5 to about 25 wt.%, preferably from about 10 to about 20 wt.%, and more preferably from about 12 to about 18 wt.%. The copolymer is preferably micronized to an average particle size less than 100, 100, or 10 microns.

Another taste masking agent is a cyclodextrin or derivative thereof. This component is preferably present in the final film in an amount of from about 10 to about 50 wt.% or, in alternative embodiments, from about 10 to about 40 wt.%, or from about 20 to about 35 wt.%.

A preferred stabilizer, especially for donepezil films, is citric acid, especially anhydrous citric acid, and in a preferred embodiment the final product comprises from about 0.5 to about 2.0 wt.% citric acid, or from about 0.75 to about 1.25 wt.% citric acid.

Alternatively, the formulation may contain one or more surfactants.

A preferred pharmaceutically active agent is ondansetron, preferably as its base. Ondansetron is chemically known as (±) 1,2,3,9 tetrahydro-9-methyl-3-[(2-methyl-1H-imidazol-1-yl)methyl]-4H-carbazol-4-one, and its base is represented by the following chemical structure:

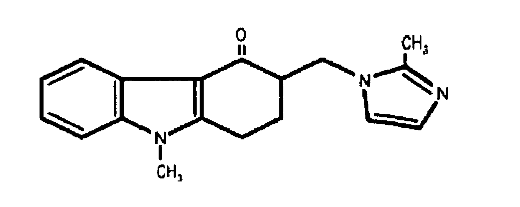

Therefore, in another embodiment the invention provides an ondansetron film strip, wherein the ondansetron is preferably provided in base form to promote GI absorption of the ondansetron. The invention also provides a non-mucoadhesive orally disintegrating film, able to disintegrate upon contact with saliva in the buccal cavity within about sixty seconds, comprising (±) 1,2,3,9 tetrahydro-9-methyl-3-[(2-methyl-IH-imidazol-I-yl)methyl]-4H-carbazol-4-one (ondansetron), in combination with a hydrophilic binder and a water-soluble diluent, and means for promoting gastrointestinal absorption of said ondansetron, wherein: (a) said means for promoting gastrointestinal absorption comprises ondansetron in base form; (b) said film comprises from about 4 to about 24 mg of ondansetron base; (c) ondansetron base is present in an amount from about 0.05% to about 50% (w/w), based on the total weight of the formulation, (d) said film has a T max of from about 1.5 to about 2.5 hours, and (e) said ondansetron base has an absolute bioavailability in said dosage form of from about 45% to about 75%. The film most preferably contains 4 or 8 mg of ondansetron base, and is preferably formulated according to the general formulation techniques described in this document.

It is known that ondansetron can exist in several polymorphic forms, including Forms A, B, C, D and E. See WO 03/093260 and WO 2005/080381. It has been unexpectedly found that the crystalline purity of the ondansetron in the final product influences the physical properties of the final film, and that highly pure form B is particularly preferred. In particular, for films stored at higher temperatures 60°C).

Physical changes in the RapidFilm have been detected, including added rigidity, warps and folding, and these changes are associated with a decrease in peak intensity and decreased purity of Form B. See Therefore, in yet another embodiment, the film comprises form B polymorph that is essentially free of other polymorphic forms, i.e. greater than 70, 80, 90, 95, 98 or even 99% pure. Form B can be evaluated by X-ray diffraction. Alternatively or in addition, the product is characterized by a melting endotherm at 244±2°C when subjected to differential scanning calorimetry.

In another embodiment, the invention provides methods of using the ondansetron film strips of the present invention, for the treatment or prevention of emesis, including emesis resulting from postoperative nausea and vomiting, chemotherapy induced nausea and vomiting, and radiation induced nausea and vomiting. Therefore, the invention also provides a method of treating or preventing emesis in a human patient comprising administering to the tongue of said patient, preferably from one to three times daily, an ondansetron film strip of the present invention that contains from about 4 to about 24 mg of ondansetron base, preferably 4 or 8 mg of ondansetron base. The method is preferably practiced with an additional step that promotes gastrointestinal absorption of said ondansetron, such as swallowing said film within about sixty seconds of said administration, with or without water.

Other film formulations of the invention are defined in international patent WO2008/040534.

### EXAMPLES

The following examples are put forth so as to provide those of ordinary skill in the art with a complete disclosure and description of how the compounds claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.) but some errors and deviations should be accounted for. Unless indicated otherwise, parts are parts by weight, temperature is in °C or is at room temperature, and pressure is at or near atmospheric.

### Example 1: Representative Ondansetron Formulation

Table 1 depicts a representative film formulation that contains 8.0 mg of ondansetron as its base, in order to promote gastrointestinal absorption.

**Table 1. Representative Formulation of Ondansetron Base Film Dosage Form**

| Pos. | Ingredient | Amount per Film [mg] | Amount per Film [%] |
|---|---|---|---|
| 1 | Ondansetron (as base) | 8.0 | 15.84 |
| 2 | Mowiol (Polyvinylalcohol) | 22.0 | 43.56 |
| 3 | PEG (polyethylene glycol) | 6.0 | 11.88 |
| 4 | Glycerol anhydrous | 2.0 | 3.96 |
| 5 | Rice Starch | 10.0 | 19.80 |
| 6 | Acesulfam K | 0.2 | 0.40 |
| 7 | Titanium dioxide | 0.3 | 0.59 |
| 8 | Menthol | 1.0 | 1.98 |
| 9 | Polysorbate | 1.0 | 1.98 |
| | TOTAL | 50.5 | 100.0 |

### Example 2 : Zophren side effects report dysphagia

Zophren Side Effects Report #5566830-8 Consumer or non-health professional from FRANCE reported Zophren side effect on Dec 10, 2007.

Male patient, 57 years of age, was diagnosed with prophylaxis of nausea and vomiting, colon cancer and was treated with Zophren. After drug was administered, patient experienced the following side effects: dysphagia, laryngeal oedema, torticollis.

### Example 3: Summary of Product Characteristics of Zophren

Ondansetron ODT (Zophran^{®} Zydis Lingual) is a dosage form intended to dissolve on the tongue. It contains ondansetron base.

The summary of product characteristics of Zophren ODT reports that in children less than 6 years of age, due to the risk of aspiration with the tablet, orally disintegrating tablet (dissolving with water) and syrup should be recommended. Therefore, Zophren tablets and ODT are not completely suitable in case of dysphagia and risk aspiration. Any of such risks has been identified with Ondansetron rapid film.

### Example 4: Comparison between Ondansetron Tablet, Ondansetron ODT and Ondansetron rapid film

In a salivary media, as defined in WO/2008/025926, and in application of a disintegration test we compare the time needed to dissolve for Zophren ODT and Zophren Rapid Film. The Zophren ODT requires between 2 and 3 minutes to dissolves, while the film of the invention containing ondansetron needs less than 60 seconds to dissolve.

## Claims

1. An oral fast dissolving film comprising an active principle for treating illnesses of patient with critical buccal condition, wherein water is not required to facilitate the dissolution or swallowing of the active principle.

2. An oral fast dissolving film according to claim 1 for treating illnesses of patient with dysphagia

3. An oral fast dissolving film according to any one of claim 1 to 2 for treating illnesses of patient presenting aspiration risks.

4. An oral fast dissolving film according to claim 1 or 3 for treating illnesses of patient with odynophagia.

5. An oral fast dissolving film according to any one of claim 1 to 4 for treating illnesses of patient with chemotherapy and/or radiotherapy-induced mucositis.

6. An oral fast dissolving film according to any one of claim 1 to 5 for treating illnesses of patient with head and neck or oesophageal cancer.

7. An oral fast dissolving film according to any one of claim 1 to 6 for treating illnesses of patient with reduced salivary flow.

8. An oral fast dissolving film according to any one of claim 1 for the treatment of dysphagia.

9. An oral fast dissolving film according to any one of claim 1 to 8 comprising an bioactive agent, a hydrophilic binder and a water-soluble diluents

10. An oral fast dissolving film according to any one of claim 1 to 9 wherein the bioactive agent is selected from: donepezil hydrochloride; ondansetron base; desloratadine; olanzapine; risperidone; rivastigmine tartrate; sildenafil; vardenafil; galantamine; diclofenac potassium; buprenorphine HCl; naloxone HCl dehydrate; alprazolam; clonazepam; diazepam; lorazepam; sumatriptan; eletriptan; rizatriptan; zolmitriptan; naratriptan; almotriptan; frovatriptan; cetirizine hydrochloride; loratadine; ambroxol hydrochloride; apomorphine; ascorbic acid; betamethasone; caffeine; dextromethorphan; glimepiride; hydrocortisone; ketotifen; loperamide; meclozine; melatonin; neramexane; piroxicam; sodium picosulfate; and zinc histidine; or a pharmaceutically acceptable salt thereof; or a combination thereof.

11. An oral fast dissolving film according to any one of claim 1 to 10 wherein the film comprises from 0.05% to 50% by weight of said bioactive agent

12. An oral fast dissolving film according to any one of claim 1 to 11 wherein the film comprises from 40% to 80% by weight of one or more ingredients that constitute a hydrophilic binder and a water soluble diluent.

13. An oral fast dissolving film according to any one of claim 1 to 12 wherein the hydrophilic binder is Polyvinylalcohol

14. An oral fast dissolving film according to any one of claim 1 to 13 wherein the water-soluble diluents is rice starch

15. An oral fast dissolving film according to any one of claim 1 to 14 comprising Ondansetron Base, Polyvinylalcohol , PolyEthylene Glycol, Glycerol anhydre, Rice starch, Polysorbate, Ethanol and Purified Water
